# EUROPÄISCHE PATENTANMELDUNG

(11) **EP 3 226 206 A1**
(43) Veröffentlichungstag der Anmeldung: **04.10.2017**
(21) Anmeldenummer: 17163292.0
(22) Anmeldetag: 28.03.2017
(51) Int. Cl.: G06T 7/00

(54) **KLASSIFIZIERUNG MITTELS AUFNAHMEN**

(71) Anmelder: Siemens Healthcare GmbH, 91052 Erlangen (DE)
(72) Erfinder: Battle, Xavier, 91301 Forchheim (DE); Hölzer, Philipp, 91088 Bubenreuth (DE); Schmidt, Bernhard, 90766 Fürth (DE); Soza, Grzegorz, 90562 Heroldsberg (DE)

(57) **Zusammenfassung**

Die Erfindung betrifft ein Verfahren zur Klassifizierung eines Untersuchungsobjekts (3) mittels Aufnahmen (A1, A2), das zumindest folgende Schritte umfasst: In einem ersten Schritt (REC) wird zumindest eine optische Aufnahme (A1) des Untersuchungsobjekts (3) erfasst. In einem zweiten Schritt werden auf Basis einer Analyse (ANA) der optischen Aufnahmen (A1) unter Verwendung eines maschinellen Lernverfahrens (L1) eine Anzahl von definierten Merkmalen (CH1, CH2,...) des Untersuchungsobjekts (3) ermittelt (DET) und quantifiziert (QUA). In einem weiteren Schritt erfolgt die Klassifizierung (CLA) des Untersuchungsobjekts (3) hinsichtlich eines Klassifizierungskriteriums (CRT) auf Basis der quantifizierten Merkmale (SC1, SC2,...) unter Verwendung eines maschinellen Lernverfahrens (L2). Des Weiteren werden eine Klassifizierungseinrichtung (20) und eine medizintechnisch bildgebende Modalität (1) beschrieben.

## Beschreibung

Die Erfindung betrifft ein Verfahren zur Klassifizierung mittels Aufnahmen, eine Klassifizierungseinrichtung und eine medizintechnisch bildgebende Modalität.

Krebserkrankungen, also maligne Tumorbildungen, werden häufig von zahlreichen Veränderungen im äußeren Erscheinungsbild eines Patienten begleitet. Diese Veränderungen werden im Rahmen einer Diagnose bisher vom medizinischen Personal lediglich qualitativ erfasst und spiegeln daher im Wesentlichen ein Gespür des Arztes für den Gesundheitszustand des Patienten wieder. Die Beurteilung des äußeren Erscheinungsbildes erfordert dabei fundierte ärztliche Erfahrung, die meist nur über langjährige Berufspraxis erarbeitet werden kann. Insbesondere bei unerfahrenem oder weniger erfahrenem Personal ist dementsprechend das Risiko für eine falsche Beurteilung verhältnismäßig hoch. Zudem stellt die rein qualitative Beurteilung des äußeren Erscheinungsbildes ein lediglich schwaches, wenig stichhaltiges Indiz für den tatsächlichen Gesundheitszustand des Patienten dar und gibt immer einen persönlichen, subjektiven Eindruck des Untersuchenden wieder.

Es ist daher eine Aufgabe der vorliegenden Erfindung, eine objektivere Klassifizierung des Gesundheitszustands eines Patienten anhand seines äußeren Erscheinungsbildes zu ermöglichen.

Diese Aufgabe wird durch ein Verfahren zur Klassifizierung gemäß Patentanspruch 1, eine Klassifizierungseinrichtung gemäß Patentanspruch 11 und eine medizintechnisch bildgebende Modalität gemäß Patentanspruch 12 gelöst.

Das eingangs genannte Verfahren zur Klassifizierung eines Untersuchungsobjekts mittels Aufnahmen umfasst zumindest folgende Schritte: In einem Schritt wird zumindest eine optische Aufnahme des Untersuchungsobjekts erfasst. In einem weiteren Schritt wird auf Basis einer Analyse der optische(n) Aufnahme(n) unter Verwendung eines maschinellen Lernverfahrens eine Anzahl von definierten Merkmalen des Untersuchungsobjekts ermittelt und quantifiziert. Die Klassifizierung des Untersuchungsobjekts hinsichtlich eines Klassifizierungskriteriums erfolgt in einem weiteren Schritt auf Basis der quantifizierten Merkmale unter Verwendung eines maschinellen Lernverfahrens.

Die Klassifizierung erfolgt bevorzugt automatisch, d. h. zumindest halbautomatisch, besonders bevorzugt vollautomatisch. Sie stellt eine Einordnung in eine bzw. Zuordnung zu einer Klasse, Kategorie oder Stufe dar. Bei dem Untersuchungsobjekt kann es sich beispielsweise um einen tierischen Patienten handeln, bevorzugt ist es jedoch ein menschlicher Patient. Im Folgenden werden daher die Begriffe "Untersuchungsobjekt" und "Patient" synonym verwendet.

Als optische Aufnahmen werden jegliche Bildaufnahmen bezeichnet, die im Wesentlichen im sichtbaren Bereich elektromagnetischer Strahlung, also in einem vom menschlichen Auge wahrzunehmenden Abschnitt des Lichtspektrums und gegebenenfalls im Infrarot- und/oder im Ultraviolettbereich, akquiriert werden. Sie werden bevorzugt ausschließlich vom Äußeren des Untersuchungsobjekts angefertigt, d. h. die Aufnahmegeräte dringen nicht in das Innere des Untersuchungsobjekts, also beispielsweise in Körperöffnungen des Patienten ein. Die Aufnahmen können dabei als ein oder mehrere Standbilder, aber auch als Bildsequenz, d. h. als Video bzw. Film des Untersuchungsobjekts, aufgezeichnet werden. Sie können sowohl zweidimensionale (2D) als auch dreidimensionale (3D) Aufnahmen umfassen. Die optischen Aufnahmen können auch eine Kombination von unterschiedlichen Aufnahmemethoden umfassen, z. B. ein hochauflösendes Standbild und ein Video mit geringerer Auflösung. Dabei kann beispielsweise der gesamte Körper eines Patienten optisch erfasst werden, es können aber auch einzelne Körperbereiche wie zum Beispiel das Gesicht und/oder die Hände separat erfasst werden, sofern es für die Ermittlung bzw. Quantifizierung des entsprechenden Merkmals zweckdienlich ist, wie später noch näher erläutert wird.

Folgend werden die Aufnahmen analysiert, wobei auch noch weitere Daten, zum Beispiel Patientendaten wie Alter, Gewicht, Größe, Vorerkrankungen und dergleichen, miteinbezogen werden können. Teilschritte der Analyse, wie beispielsweise eine Bereichsauswahl oder das Setzen von Parametern, können von einem Bediener vorgenommen werden, bevorzugt erfolgt die Analyse jedoch vollautomatisch mittels eines maschinellen Lernverfahrens, wie später noch näher beschrieben wird. Im Rahmen der Analyse können in Abhängigkeit von dem maschinellen Lernverfahren einzelne Merkmale zunächst separat in einem Teilschritt ermittelt und dann in einem weiteren Teilschritt quantifiziert werden, das Ermitteln und das Quantifizieren können aber auch in nur einem Schritt, d. h. gleichzeitig und gemeinsam bzw. simultan erfolgen.

Im Rahmen der Analyse können markante topographische Eigenschaften als Merkmale zunächst festgestellt und folgend zueinander in Bezug gesetzt werden. Zum Beispiel können anatomische Landmarken ermittelt und bezüglich ihrer Distanz und/oder Anordnung ausgewertet werden. Weiterhin können beispielsweise die Ausdehnung bzw. das Volumen von Körperteilen, Organen und/oder Extremitäten bestimmt werden und in die Analyse miteinfließen. So können zum Beispiel Körperproportionen ermittelt und in Relation zueinander ausgewertet werden. Alternativ oder zusätzlich kann dabei auch eine farbliche Erfassung, insbesondere der Hautfarbe bzw. Farbe der Bindehaut, vorgenommen werden.

Die Quantifizierung der Merkmale bezeichnet eine Repräsentation des Merkmals als Zahlenwert. Sie kann dabei in Abhängigkeit von dem jeweiligen Merkmal beispielsweise durch absolute Parameter oder durch relative Parameter erfolgen. Als absolute Parameter können zum Beispiel messbare Größen wie Länge, Dicke, Volumen, Form, geometrische Anordnung und dergleichen von Körperteilen bzw. Organen des Patienten erfasst werden. Mit Hilfe von Videoaufnahmen kann in einem weiteren Beispiel die Zeit zwischen Atemzügen oder Hustenbewegungen (bzw. in Abhängigkeit von der jeweiligen Anwendung die Frequenz) als absoluter Parameter gemessen werden. Die absoluten Parameter können also im Wesentlichen direkt quantifiziert werden. Dabei können unterschiedliche Parameter aber auch zueinander ins Verhältnis gesetzt und so als neuer - ggf. auf ein anderes Merkmal bezogener - Parameter ausgewertet werden. So kann beispielsweise die Farbe als absoluter Parameter oder im Verhältnis gemessen werden. Bei als absoluten Parametern gemessenen Farbwerten wird die Analyse bevorzugt direkt auf deren Basis durchgeführt, andernfalls werden beispielsweise die Farben unterschiedlicher Körperregionen des Patienten zueinander in ein Verhältnis gesetzt.

Relative Parameter - z. B. ein relatives Maß für die Verwirrtheit des Patienten - können im Allgemeinen nicht direkt gemessen werden. Die entsprechenden Merkmale können in diesem Fall beispielsweise durch eine komplexe Analyse des gesamten Erscheinungsbildes des Patienten beurteilt werden. Sie werden daher beispielsweise durch eine Bewertung auf einer Skala bzw. anhand eines Bewertungsmaßstabes quantifiziert. Im Gegensatz zur bisherigen Beurteilung durch einen Arzt werden erfindungsgemäß mittels des Quantifizierens somit die - ggf. abstrakten - Merkmale in Parameter mit zugeordneten Parameterwerten transformiert und so messbar bzw. auswertbar gemacht.

Die Klassifizierung bezeichnet eine komplexe Zusammenschau auf Basis einer Auswertung der quantifizierten Merkmale hinsichtlich des Klassifizierungskriteriums. Je nach Klassifizierungskriterium können die einzelnen Merkmale dafür beispielsweise unterschiedlich gewichtet oder in unterschiedliche Abhängigkeiten zueinander gesetzt werden. D. h. wenn zum Beispiel ein definiertes Merkmalspaar oder eine definierte Merkmalsgruppe in besonders starker Ausprägung auftritt, ist sie auch in besonderem Maße relevant für die Klassifizierung. Das Klassifizierungskriterium ist dabei bevorzugt ein definiertes Krankheitsbild. Die Klassifizierung gibt somit bevorzugt an, ob das Krankheitsbild auf den Patienten zutrifft oder nicht. Auch die Klassifizierung erfolgt unter Verwendung eines maschinellen Lernverfahrens. Dabei kann ein erstes maschinelles Lernverfahren für die Analyse, d. h. die Ermittlung beziehungsweise die Quantifizierung der Merkmale, und im Unterschied dazu ein zweites maschinelles Lernverfahren für die Klassifizierung verwendet werden. Auch können je nach Bedarf für unterschiedliche zu ermittelnde und zu quantifizierende Merkmale unterschiedliche maschinelle Verfahren eingesetzt werden. Es kann aber auch in Abhängigkeit von der jeweiligen Anwendung teilweise oder insgesamt das gleiche maschinelle Lernverfahren eingesetzt werden. Das erfindungsgemäße Verfahren ermöglicht dadurch eine objektivere, einfachere und schnellere Einordnung hinsichtlich des Klassifizierungskriteriums.

Die eingangs genannte Klassifizierungseinrichtung weist zur Klassifizierung eines Untersuchungsobjekts mittels Aufnahmen eine Erfassungseinheit, eine Analyseeinheit und eine Klassifizierungseinheit auf. Sie ist dabei so ausgebildet, dass sie die Schritte eines erfindungsgemäßen Verfahrens zur Klassifizierung eines Untersuchungsobjekts ausführt. Die Analyseeinheit umfasst dabei bevorzugt eine Ermittlungseinheit und eine Quantifizierungseinheit. Die Ermittlungseinheit ist dabei bevorzugt so ausgebildet, dass sie im Rahmen der Analyse die (optischen) Aufnahmen des Untersuchungsobjekts auswertet und daraus, wie zuvor bereits beschrieben, definierte Merkmale bestimmt. Die Quantifizierungseinheit ist so ausgebildet, dass sie die definierten Merkmale folgend oder auch simultan zur Ermittlung quantifiziert.

Die eingangs genannte medizintechnisch bildgebende Modalität umfasst eine erfindungsgemäße Klassifizierungseinrichtung. Die medizintechnisch bildgebende Modalität kann beispielsweise als MRT-System (Magnetresonanztomograph), Angiographiesystem, Ultraschallsystem oder PET-System (Positronenemissionstomograph) ausgebildet sein. Bevorzugt ist sie jedoch als CT-System (Computertomograph) ausgebildet. Das Zusammenwirken der erfindungsgemäßen Klassifizierungseinrichtung mit den übrigen Komponenten der medizintechnisch bildgebenden Modalität ist dabei besonders vorteilhaft, da auf diese Weise Informationen einerseits über das Innere des Patienten und andererseits über sein äußeres Erscheinungsbild gewonnen werden können. Es können somit also beispielsweise sowohl die Entwicklung von Karzinomen während einer Behandlung als auch weitere äußerliche Symptome der Erkrankung quantitativ erfasst werden.

Die wesentlichen Komponenten der erfindungsgemäßen Klassifizierungseinrichtung können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Insbesondere kann die erfindungsgemäße Klassifizierungseinrichtung Teil eines Benutzerterminals einer medizintechnisch bildgebenden Modalität sein.

Eine weitgehend softwaremäßige Realisierung hat den Vorteil, dass auch schon bisher verwendete Benutzerterminals bzw. Rechnereinheiten auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Klassifizierungseinrichtung einer medizintechnisch bildgebenden Modalität ladbar ist, mit Programmabschnitten, um alle Schritte des erfindungsgemäßen Verfahrens auszuführen, wenn das Programm in der Klassifizierungseinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Klassifizierungseinrichtung und/oder zur Speicherung an oder in der Klassifizierungseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Klassifizierungseinrichtung einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die unabhängigen Ansprüche einer Anspruchskategorie auch analog zu den abhängigen Ansprüchen oder Beschreibungsteilen einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Vorzugsweise wird das Untersuchungsobjekt hinsichtlich einer Krebserkrankung klassifiziert. Das heißt, als Klassifizierungskriterium dient bevorzugt eine Krebserkrankung, also eine bösartige Gewebeneubildung bzw. ein maligner Tumor. Bei der Krebserkrankung, hinsichtlich derer klassifiziert werden soll, handelt es sich besonders bevorzugt um Lungenkrebs. Dieser tritt häufig in Kombination mit den im Folgenden beschriebenen Symptomen auf.

Die definierten, zu ermittelnden und zu quantifizierenden Merkmale umfassen bei einem erfindungsgemäßen Verfahren vorzugsweise zumindest ein Merkmal der folgenden Liste: Bindehautblässe, Gelbsucht, Trommelschlägelfinger, Horner-Syndrom, Cushing-Syndrom, andauernder Husten, Hämoptyse, Atemlosigkeit, Dysphagie, Verwirrung, Anorexie, Müdigkeit und/oder Aszites.

Als Trommelschlägelfinger wird dabei eine Auftreibung bzw. Verdickung von Finger- und Zehengliedern bezeichnet. Das Horner-Syndrom ist eine spezifische Form einer Nervenschädigung, die durch einen Ausfall des Sympathikuskopfteils verursacht wird. Es weist einen zumeist einseitig vorkommenden, dreiteiligen Symptomkomplex auf, der aus einer Pupillenverengung, dem Herabhängen eines Oberlids und einem gering in die Augenhöhle eingesunkenen Augapfel besteht. Das Cushing-Syndrom bezeichnet bekanntermaßen körperliche Veränderungen, die durch einen hohen Cortisolspiegel im Blut verursacht werden, wobei sich typischerweise am Körperstamm vermehrt Fettgewebe ansammelt und die Gliedmaßen durch Muskelschwund dünner werden. Auch die übrigen gelisteten Merkmale sind symptomatisch bekannt. So bezeichnet die Hämoptyse einen Bluthusten, die Dysphagie eine Schluckstörung, Aszites bezeichnet Bauchwassersucht und Anorexie bezeichnet Appetitlosigkeit.

Einige Merkmale können dabei absolut in Parameterwerten gemessen werden, wie zum Beispiel das Volumen von Finger- und Zehengliedern beim Merkmal "Trommelschlägelfinger". Andere Merkmale können im Vergleich zu anderen Körperbereichen, also beispielsweise durch einen Vergleich der Gesichtshälften beim Horner-Syndrom, ermittelt und quantifiziert werden. Bei wiederum anderen Merkmalen, wie zum Beispiel der Verwirrung bzw. Verwirrtheit des Patienten, ist eine komplexe Analyse der optischen und ggf. akustischen Aufnahmen erforderlich.

Grundsätzlich können beliebige Merkmale aus der Liste in die Klassifizierung miteinfließen. Das heißt, je nach Bedarf kann beispielsweise nur eine ausgewählte Gruppe von Merkmalen verwendet werden. Besonders bevorzugt erfolgt die Klassifizierung jedoch unter Verwendung aller Merkmale, die in der Liste aufgeführt sind.

Bei einem erfindungsgemäßen Verfahren wird bevorzugt zusätzlich eine Anzahl von akustischen Aufnahmen des Untersuchungsobjekts erfasst, welche mit zur Klassifizierung genutzt werden. Die akustischen Aufnahmen können beispielsweise mittels eines Mikrofons erfasst werden. Ihre zusätzliche Verwendung erleichtert vorteilhafterweise zum Beispiel die Ermittlung und Quantifizierung von andauerndem Husten und/oder Atemlosigkeit.

Die akustischen Aufnahmen werden besonders bevorzugt mittels einer Sprachanalyse analysiert. Dadurch können sie beispielsweise auch Aufschluss über die Verwirrtheit des Patienten geben, wenn unzusammenhängende Worte oder undeutliches Vorsichhinreden (Gebrabbel) erfasst werden. Die Nutzung zur Klassifizierung bedeutet also vorzugsweise, dass die bereits zuvor beschriebenen, mit den optischen Aufnahmen durchgeführten Verfahrensschritte auch mit den akustischen Aufnahmen durchgeführt werden.

Bevorzugt umfassen die maschinellen Lernverfahren bei einem erfindungsgemäßen Verfahren zumindest einen der folgenden Algorithmen: Support Vector Machines, Bayes-Klassifikator (Bayesian classifiers), k-Means-Algorithmus (k-means clustering), Deep-Belief-Netzwerk (deep belief networks), tiefes Residuallernen (deep residual learning), bestärkendes Lernen (reinforcement learning), Entscheidungsbäume (decision trees), rekurrente neuronale Netze (recurrent neural networks), induktives Programmieren (inductive programming) oder bevorzugt faltende neuronale Netze (convolutional neural networks). Die genannten Algorithmen und ihre jeweilige Funktionsweise sind dabei aus folgenden Veröffentlichungen bekannt, deren Inhalte hier insoweit vollumfänglich inkorporiert werden:
- "An Introduction to Machine Learning", Miroslav Kubat, Springer
- "Deep Learning", Goodfellow, Bengio, Courville, MIT Press
- "Machine learning: Trends, perspectives, and prospects", Jordan, Mitchell, Science 2015(349) 255.

Vor der Durchführung eines erfindungsgemäßen Verfahrens wird der Algorithmus bzw. werden die Algorithmen bevorzugt mithilfe eines Trainingsverfahrens vorbereitet. Dabei lernen die Algorithmen anhand von vorgehaltenen bzw. bereitgestellten Trainingsdaten, die von früheren Patienten akquiriert wurden, wie die definierten Merkmale zu ermitteln bzw. zu quantifizieren sind. Zugleich oder separat lernen sie, wie folgend auf Basis der Analyse dieser Merkmale die Klassifizierung hinsichtlich des Klassifizierungskriteriums vorgenommen wird. Gegebenenfalls können das Ermitteln und das Quantifizieren für ein einzelnes geeignetes Merkmal auch separat trainiert werden, soweit für dieses Merkmal Vorratsdaten bzw. Standardbilder (stock photography) vorliegen.

Das Training bzw. das Trainingsverfahren wird bevorzugt bei wiederholter Durchführung des erfindungsgemäßen Verfahrens iterativ fortgesetzt (inline machine learning), sodass mit steigender Anzahl der durchgeführten Verfahren bzw. steigender Anzahl der klassifizierten Untersuchungsobjekte die Ergebnisse des Verfahrens immer weiter verbessert werden. Die Trainings- bzw. Klassifizierungsdaten werden bevorzugt zwischen unterschiedlichen Untersuchungsorten bzw. unterschiedlichen erfindungsgemäß arbeitenden, medizintechnisch bildgebenden Modalitäten zum Beispiel über ein Netzwerk (cloud) ausgetauscht. Dadurch wird vorteilhafterweise eine Beschleunigung des Lernprozesses und somit eine schnellere Verbesserung der erfindungsgemäßen Klassifizierung erzielt. Im Rahmen des Trainings - also auch nach jeder Durchführung des erfindungsgemäßen Verfahrens - kann hierfür besonders bevorzugt eine Beurteilung bzw. Bewertung der Klassifizierung durch einen Bediener vorgenommen werden, die als Rückkopplung (feedback) für das Training zur Verbesserung der Ergebnisse verwendet wird.

Bevorzugt wird bei einem erfindungsgemäßen Verfahren zusätzlich zur Klassifizierung eine Wahrscheinlichkeit einer richtigen Klassifizierung ermittelt. Das heißt, neben der Klassifizierung an sich wird bevorzugt auch analysiert, mit welcher Sicherheit richtig klassifiziert wurde. Die Wahrscheinlichkeit kann dabei auch als Wert ausgegeben werden, bei dem es sich beispielsweise um ein Konfidenzintervall und/oder eine prozentuale Angabe handeln kann. Dadurch wird vorteilhafterweise nicht nur eine objektivere Klassifizierung erreicht, sondern auch angegeben, wie vertrauenswürdig die Klassifizierung zu beurteilen ist.

Wie eingangs bereits erwähnt, ergeben sich besondere synergetische Effekte, wenn sowohl das äußere Erscheinungsbild eines Patienten als auch Daten von seinem Inneren hinsichtlich des Klassifizierungskriteriums beurteilt werden. Daher wird das erfindungsgemäße Verfahren bevorzugt mit einer Untersuchung mittels einer medizintechnisch bildgebenden Modalität verknüpft, welche besonders bevorzugt Daten vom Inneren des Untersuchungsobjekts erfasst.

Die optischen Aufnahmen werden vorzugsweise mit Hilfe von einer 2D-Kamera, einer 2D-RGB-Kamera und/oder einer 3D-Kamera aufgenommen. Dabei können je nach Bedarf günstige, handelsübliche Kameras bzw. Sensoren oder hochwertige Modelle eingesetzt werden, die vergleichsweise bessere Daten akquirieren und somit auch eine bessere Analyse ermöglichen. Mithilfe der Kameras können also beispielsweise Graustufenwerte oder Farbwerte des Untersuchungsobjekts erfasst werden, es können aber auch Falschfarbenaufnahmen, wie zum Beispiel Infrarotbilder, oder ein räumlicher Eindruck bzw. eine Entfernungsmessung mittels einer 3D-Kamera akquiriert werden. Als 3D-Kamera werden dabei unterschiedliche Systeme wie beispielsweise eine Stereokamera, TOF-Kameras (time of flight) oder auch Triangulationssysteme, die Verzerrungen eines definierten Lichtmusters auf dem Untersuchungsobjekt vermessen, verstanden.

Bevorzugt umfasst das Ermitteln der definierten Merkmale die Verwendung eines Gesichtserkennungsalgorithmus. Gesichtserkennung bezeichnet dabei eine Analyse der Ausprägung sichtbarer Merkmale im Bereich des frontalen Kopfes des Patienten, gegeben durch geometrische Anordnung und Textureigenschaften der Oberfläche. Derartige Algorithmen sind bereits detailliert ausgearbeitet, frei zugänglich (open source) sowie ressourceneffizient und können somit vorteilhaft im Rahmen des erfindungsgemäßen Verfahrens eingesetzt werden.

Bevorzugt wird mit Hilfe des erfindungsgemäßen Verfahrens die Veränderung des Untersuchungsobjekts hinsichtlich des Klassifizierungskriteriums zwischen einer ersten Untersuchung und zumindest einer Folgeuntersuchung ermittelt. Dadurch kann beispielsweise der Krankheitsverlauf und/oder das Ansprechen auf eine bestimmte Therapie bei einem Patienten dokumentiert werden. Insbesondere können dabei die Veränderungen der Merkmale im Zeitraum zwischen den Untersuchungen in Relation zueinander analysiert werden. Das heißt, dass die Merkmale bei den Folgeuntersuchungen in Relation zur ersten Untersuchung beurteilt bzw. quantifiziert werden können.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
FIG 1 eine schematische Ansicht eines Ausführungsbeispiels einer erfindungsgemäßen medizintechnisch bildgebenden Modalität,
FIG 2 ein Blockschema eines Ausführungsbeispiels eines erfindungsgemäßen Verfahrens zur Klassifizierung,
FIG 3 ein Blockschema eines weiteren Ausführungsbeispiels eines erfindungsgemäßen Verfahrens zur Klassifizierung und
FIG 4 eine schematische Ansicht einer optischen Aufnahme eines Untersuchungsobjekts.

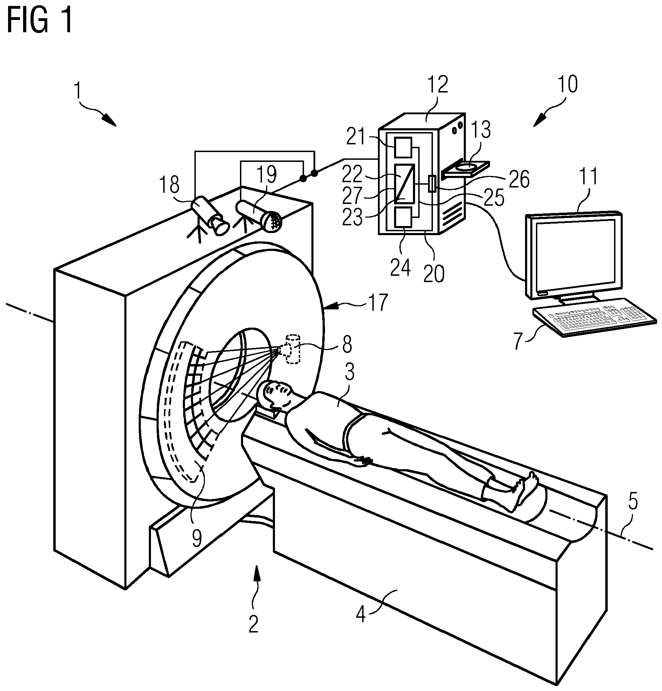

In FIG 1 ist beispielhaft und grob schematisch ein Computertomographiesystem 1 als erfindungsgemäße medizintechnisch bildgebende Modalität 1 gezeigt, welches ein Benutzerterminal 10 und ein Computertomographiegerät 2 umfasst. Das Computertomographiesystem 1 ist zur Ausführung des erfindungsgemäßen Verfahrens zur Klassifizierung ausgebildet. Das Computertomographiegerät 2 umfasst einen Patiententisch 4 zur Lagerung eines Patienten 3 als Untersuchungsobjekt 3, welcher entlang einer Systemachse 5 verstellbar ist. Die Systemachse 5 wird im Folgenden auch als z-Achse bezeichnet, entlang derer der Patienten 3 in das Messfeld positionierbar ist. Es umfasst ferner eine Gantry 17 mit einer um die Systemachse 5 drehbar gelagerten Quelle-Detektor-Anordnung 8, 9. Die Quelle-Detektor-Anordnung 8, 9 weist eine Röntgenstrahlungsquelle 8 und einen Detektor 9 auf, die einander gegenüberliegend so ausgerichtet sind, dass im Betrieb eine von dem Fokus der Röntgenstrahlungsquelle 8 ausgehende Röntgenstrahlung auf den Detektor 9 trifft. Die Funktionsweise eines solchen CT-Geräts 2 ist grundsätzlich bekannt und wird daher an dieser Stelle nicht weiter ausgeführt.

Das Computertomographiesystem 1 umfasst zusätzlich eine Kamera 18, z. B. eine 3D-Stereokamera (hier schematisch dargestellt) zur Akquisition von dreidimensionalen optischen Aufnahmen A1 und ein Mikrofon 19 zur Akquisition von akustischen Aufnahmen A2. Das Benutzerterminal 10 umfasst eine Rechnereinheit 12, eine Anzeigeeinheit 11, beispielsweise einen Bildschirm, und eine Eingabeeinheit 7, beispielsweise eine Tastatur, zum Erfassen von Benutzereingaben. Die Rechnereinheit 12 umfasst eine erfindungsgemäße Klassifizierungseinrichtung 20 sowie ein Laufwerk 13 zum Einlesen eines erfindungsgemäßen computerlesbaren Mediums. Die Klassifizierungseinrichtung 20 ist dabei zur Ausführung des erfindungsgemäßen Verfahrens ausgebildet. Das Ergebnis des erfindungsgemäßen Verfahrens, also die Klassifizierung, ist z. B. auf der Anzeigeeinheit 11 darstellbar und/oder kann in einem Speicher hinterlegt werden und/oder an andere Systeme übermittelt werden. So kann die Klassifizierung für eine folgende Diagnose durch medizinisches Personal, als Vergleich bei Folgeuntersuchungen und/oder zum Training eines maschinellen Lernverfahrens genutzt werden.

Die Klassifizierungseinrichtung 20 ist beispielhaft in einem Blockschaltbild dargestellt. Sie umfasst eine Schnittstelle 26, eine Erfassungseinheit 21, eine Analyseeinheit 27 mit einer Ermittlungseinheit 22 und einer Quantifizierungseinheit 23 sowie eine Klassifizierungseinheit 24, welche mittels eines Busses 25 zur Datenübertragung verbunden sind. Zwischen den Komponenten der Analyseeinheit 27 können Daten über den Bus 25 also frei ausgetauscht werden. Mittels der Schnittstelle 26 ist die Klassifizierungseinrichtung 20 z. B. mit einem Netzwerk, Speichereinrichtungen und/oder mit anderen Komponenten des CT-Systems 1 wie beispielsweise der 3D-Kamera 18 und dem Mikrofon 19 verbunden. Sie dient der Datenübertragung von diesen Komponenten zu der Klassifizierungseinrichtung 20 und ggf. auch umgekehrt.

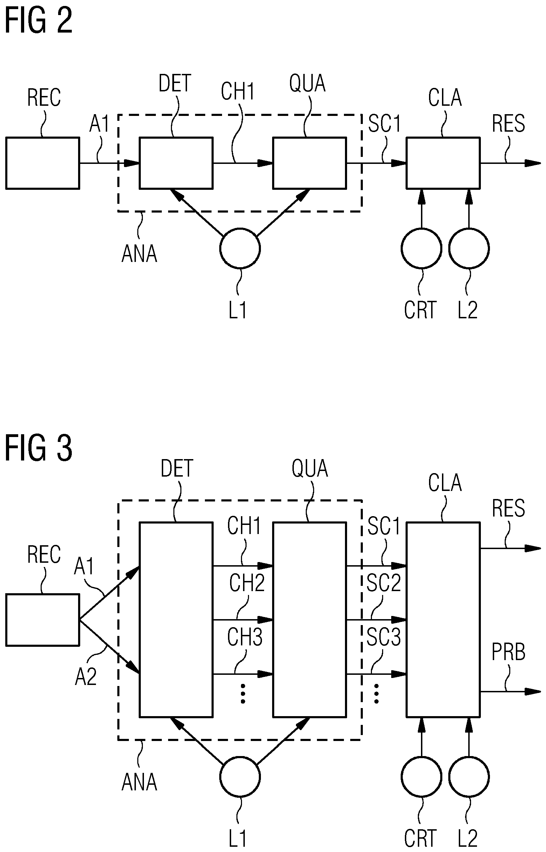

FIG 2 zeigt ein Ausführungsbeispiel eines erfindungsgemäßen Verfahrens zur Klassifizierung in einer blockschematischen Darstellung. In einem Schritt REC werden dreidimensionale optische Aufnahmen A1 - z. B. 3D-Videoaufnahmen - mit Hilfe der Stereokamera 18 von dem Patienten 3 erfasst und über die Schnittstelle 26 der Klassifizierungseinrichtung 20 an deren Erfassungseinheit 21 übertragen und dort beispielsweise zwischengespeichert. In einem folgenden Schritt ANA, der hier in zwei Unterschritte DET, QUA untergliedert ist, werden die erfassten Daten bzw. die Aufnahmen A1 in der Analyseeinheit 27 mit Hilfe eines zuvor anhand von anderen Patientendaten bzw. Patientenaufnahmen trainierten maschinellen Lernverfahrens L1 - hier z. B. "convolutional neural networks" - ausgewertet.

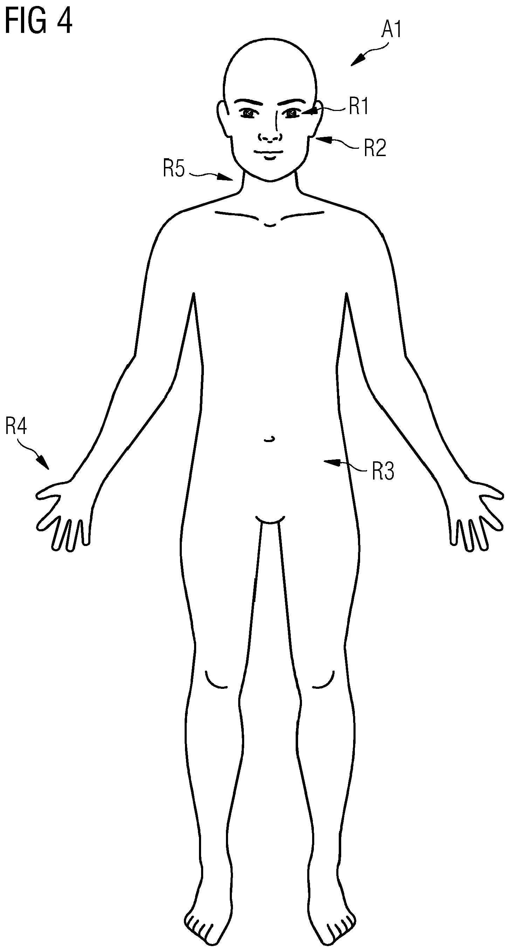

Die Unterschritte DET, QUA der Analyse ANA können dabei in Abhängigkeit des betrachteten definierten Merkmals CH1 gegebenenfalls ineinandergreifen bzw. eng miteinander verwoben sein. Die definierten Merkmale CH1, CH2, CH3,... werden folgend kurz anhand von FIG 4 beschrieben.

In FIG 4 ist eine optische Aufnahme A1 einer Frontalansicht eines Patienten 3 schematisch dargestellt. Der Patient weist eine Anzahl von Körperregionen R1, R2, ..., R5 auf, wobei die Körperregionen R1, R2, ..., R5 unterschiedlich relevant für die Analyse eines Merkmals sein können. So wird beispielsweise zur Ermittlung und Quantifizierung hinsichtlich der Merkmale "Bindehautblässe" und "Horner-Syndrom" im Wesentlichen eine Augenregion R1 des Patienten 3 analysiert. Wobei zur Feststellung und Bewertung der Bindehautblässe zum Beispiel ein absoluter Farbabgleich erfolgt und beim Horner-Syndrom beide Gesichtshälften miteinander verglichen werden. Hinsichtlich des Merkmals "Gelbsucht" kann beispielsweise eine Analyse einer Gesichtsfarbe in einer Gesichtsregion R2, also auch der Augenregion R1 und hier insbesondere der Lederhaut des Auges, erfolgen. Zur Analyse des Merkmals "Trommelschlägelfinger" wird insbesondere eine Handregion R4 betrachtet und hier das Volumen bzw. die räumlichen Abmessungen von Fingern und Händen beurteilt und beispielsweise in Relation zu einem Körpergewicht des Patienten 3 gesetzt.

Zur Analyse des Merkmals "Aszites" wird beispielsweise in einer Bauchregion R3 ein Bauchumfang bzw. ein Volumen des Bauches des Patienten ermittelt und in Relation zu seinen übrigen Körpermaßen bewertet. In ähnlicher Weise kann das Merkmal "Anorexie" analysiert werden, wobei hier auch eine Gesamtbetrachtung der Aufnahmen A1 des Patienten 3 Aufschluss über eine Abmagerung geben und in die Analyse mit einfließen kann.

Die Merkmale "Dysphagie", "andauernder Husten" und "Hämoptyse" können beispielsweise mittels einer Analyse einer Halsregion R5 ermittelt und quantifiziert werden, wobei hier eine zeitliche Betrachtung der Halsregion R5 im Hinblick auf unregelmäßige Bewegungsabläufe die nötigen Informationen liefern kann. Bei der Hämoptyse kann zusätzlich auch die Farbe des Auswurfs in Betracht gezogen werden. Auch können zur Analyse dieser Merkmale zusätzlich akustische Aufnahmen A2 des Patienten 3 genutzt werden, wie später noch näher erläutert wird.

Eine Ermittlung beziehungsweise Bewertung des Merkmals "Cushing-Syndrom" kann beispielsweise über eine Analyse der geometrischen Formen im Gesichtsbereich R2 und im Halsbereich R5 des Patienten erfolgen. Für die Merkmale "Atemlosigkeit", "Verwirrung" und "Müdigkeit" kann eine Gesamtbetrachtung aller Körperregionen R1, R2, ..., R5 des Patienten 3 erforderlich sein, da hier das Erscheinungsbild beziehungsweise das Verhalten des Patienten insgesamt maßgeblich ist.

Zusammenfassend können die definierten Merkmale also zum Beispiel durch Analyse der Länge, der Dicke, des Volumens, der Form, der geometrischen Anordnung sowie der Farbe von Regionen des Patientenkörpers oder auch in einer Zusammenschau oder einer zeitlichen Veränderung dieser Parameter ermittelt und/oder quantifiziert werden. Dabei ist auch eine Kombination der optischen Aufnahmen A1 mit akustischen Aufnahmen für die Analyse bei einigen definierten Merkmalen zweckdienlich.

Diese komplexe Analyse wird insbesondere durch die Verwendung eines maschinellen Lernverfahrens L1 ermöglicht (siehe FIG 2). Dabei kann ein definiertes Merkmal CH1 zunächst in einem ersten Zwischenschritt DET in der Ermittlungseinheit 22 ermittelt und folgend in einem weiteren Zwischenschritt QUA in der Quantifizierungseinheit 23 quantifiziert werden. Die Ermittlung DET und die Quantifizierung QUA können aber auch in einem einzigen Analyseschritt ANA simultan erfolgen. Entsprechend können Ermittlungseinheit 22 und Quantifizierungseinheit 23 zusammen in einer Einheit ausgebildet sein. Als Ergebnis der Analyse ANA wird jedenfalls zumindest ein quantifiziertes Merkmal SC1 erhalten.

Auf Basis des quantifizierten Merkmals SC1 wird in einem folgenden Schritt CLA der Patient 3 hinsichtlich eines Klassifizierungskriteriums CRT, also beispielsweise hinsichtlich einer Lungenkrebserkrankung, klassifiziert. Die Klassifizierung CLA erfolgt dabei unter Verwendung eines zweiten maschinellen Lernverfahrens L2, wobei auch hier bevorzugt "convolutional neural networks" eingesetzt werden. Ein Ergebnis RES der Klassifizierung CLA, also z. B. die Einordnung zu oder entgegen einer Lungenkrebserkrankung, kann beispielsweise auf dem Bildschirm 11 des Benutzerterminals 10 ausgegeben werden oder in einer Speichereinrichtung und/oder einem Netzwerk hinterlegt werden, die mit der Klassifizierungseinrichtung 20 verbunden sind.

Das in FIG 3 blockschematisch dargestellte Ausführungsbeispiel eines erfindungsgemäßen Verfahrens zur Klassifizierung ist im Wesentlichen ähnlich zu dem anhand von FIG 2 beschriebenen Verfahren. Im Unterschied dazu werden hier jedoch sowohl optische Aufnahmen A1 als auch akustische Aufnahmen A2 des Patienten 3 erfasst. Aus diesen Aufnahmen A1, A2 werden in dem folgenden Analyseschritt ANA definierte Merkmale CH1, CH2, CH3,... ermittelt und quantifiziert, wobei in diesem Schritt zusätzlich zu den optischen Aufnahmen A1 auch die akustischen Aufnahmen A2 für die Analyse genutzt werden. Auch diese komplexe Analyse erfolgt bevorzugt unter Verwendung von "convolutional neural networks" als maschinellem Lernverfahren L1. Zu jedem definierten Merkmal CH1, CH2, CH3,... wird mittels der Analyse ein quantifiziertes Merkmal SC1, SC2, SC3,... erzeugt, wie für die einzelnen Merkmale anhand von FIG 4 bereits beschrieben wurde.

Aus den quantifizierten Merkmalen SC1, SC2, SC3,... wird im folgenden Klassifizierungsschritt CLA sowohl ein Ergebnis RES der Klassifizierung CLA, d. h. die Klassifizierung an sich, als auch eine Wahrscheinlichkeit PRB für die Richtigkeit der Klassifizierung, d. h. beispielsweise ein Konfidenzintervall, ermittelt. Da die Auswertung der quantifizierten Merkmale SC1, SC2, SC3,... eine komplexe Bewertung in ihrer Zusammenschau erfordert, werden zur Klassifizierung CLA bevorzugt wieder "convolutional neural networks" als zweites maschinelles Lernverfahren L2 eingesetzt. Dabei können die einzelnen quantifizierten Merkmale beispielsweise unterschiedlich gewichtet und/oder in komplexe Abhängigkeiten zueinander gesetzt werden. Zum Beispiel kann ein Merkmal vollkommen ignoriert werden, wenn sich ein anderes Merkmal innerhalb eines bestimmten Wertebereiches befindet und/oder Merkmale können sich bei einem gemeinsamen Auftreten in ihrem Einfluss auf die Klassifizierung verstärken.

Die mittels des erfindungsgemäßen Verfahrens erhaltenen Ergebnisse RES, PRB können für sich bereits ein Indiz für eine Diagnose durch medizinisches Personal darstellen, bevorzugt werden sie jedoch in Kombination mit den Ergebnissen verwendet, die aus einer Untersuchung bzw. Bildgebung mithilfe der medizintechnisch bildgebenden Modalität 1, also dem CT-System 1, gewonnen wurden. Die Ergebnisse können zusätzlich in einem Speicher hinterlegt werden und so bei Folgeuntersuchungen den Krankheitsverlauf bzw. den Erfolg einer Therapie dokumentieren. Insgesamt wird durch das erfindungsgemäße Verfahren eine objektivere Bewertung des äußeren Erscheinungsbildes des Patienten hinsichtlich seiner Erkrankung ermöglicht.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einrichtung", "Einheit" und "System" nicht aus, dass die betreffende Komponente aus mehreren zusammenwirkenden Teilkomponenten besteht, die gegebenenfalls auch räumlich verteilt sein können. So können beispielsweise ein CT-Gerät und eine Klassifizierungseinrichtung einer erfindungsgemäßen medizintechnisch bildgebenden Modalität einerseits zwar in einem einzigen Raum angeordnet sein, sie können jedoch andererseits auch über weite Distanzen über ein Netzwerk miteinander verbunden sein.

## Patentansprüche

1. Verfahren zur Klassifizierung eines Untersuchungsobjekts (3) mittels Aufnahmen (A1, A2) umfassend zumindest folgende Schritte:
- Erfassen (REC) von zumindest einer optischen Aufnahme (A1) des Untersuchungsobjekts (3),
- Ermitteln (DET) einer Anzahl von definierten Merkmalen (CH1, CH2,...) des Untersuchungsobjekts (3) und Quantifizieren (QUA) der Merkmale (CH1, CH2,...) auf Basis einer Analyse (ANA) der optischen Aufnahmen (A1) unter Verwendung eines maschinellen Lernverfahrens (L1) und
- Klassifizierung (CLA) des Untersuchungsobjekts (3) hinsichtlich eines Klassifizierungskriteriums (CRT) auf Basis der quantifizierten Merkmale (SC1, SC2,...) unter Verwendung eines maschinellen Lernverfahrens (L2).

2. Verfahren nach Anspruch 1, wobei das Untersuchungsobjekt (3) hinsichtlich einer Krebserkrankung klassifiziert wird.

3. Verfahren nach Anspruch 2, wobei es sich bei der Krebserkrankung um Lungenkrebs handelt.

4. Verfahren nach Anspruch 3, wobei die definierten Merkmale (CH1, CH2,...) zumindest ein Merkmal der folgenden Liste umfassen: Bindehautblässe, Gelbsucht, Trommelschlägelfinger, Horner-Syndrom, Cushing-Syndrom, andauernder Husten, Hämoptyse, Atemlosigkeit, Dysphagie, Verwirrung, Anorexie, Müdigkeit und/oder Aszites.

5. Verfahren nach einem der vorstehenden Ansprüche, wobei zusätzlich eine Anzahl von akustischen Aufnahmen (A2) des Untersuchungsobjekts (3) erfasst wird und die akustischen Aufnahmen mit zur Klassifizierung (CLA) genutzt werden.

6. Verfahren nach einem der vorstehenden Ansprüche, wobei die maschinellen Lernverfahren (L1, L2, L3,...) zumindest einen der folgenden Algorithmen umfassen: Support Vector Machines, Bayes-Klassifikator, k-Means-Algorithmus, Deep-Belief-Netzwerk, tiefes Residuallernen, bestärkendes Lernen, Entscheidungsbäume, rekurrentes neuronales Netz, induktives Programmieren oder bevorzugt faltendes neuronales Netz.

7. Verfahren nach einem der vorstehenden Ansprüche, wobei zusätzlich zur Klassifizierung (CLA) eine Wahrscheinlichkeit (PRB) einer richtigen Klassifizierung ermittelt wird.

8. Verfahren nach einem der vorstehenden Ansprüche, wobei das Verfahren mit einer Untersuchung mittels einer medizintechnisch bildgebenden Modalität (1) verknüpft wird.

9. Verfahren nach einem der vorstehenden Ansprüche, wobei die optischen Aufnahmen (A1) mit Hilfe einer 2D-Kamera (18), einer 2D-RGB-Kamera (18) und/oder einer 3D-Kamera (18) aufgenommen werden.

10. Verfahren nach einem der vorstehenden Ansprüche, wobei das Ermitteln der definierten Merkmale (CH1, CH2,...) die Verwendung eines Gesichtserkennungsalgorithmus umfasst.

11. Klassifizierungseinrichtung (20) zur Klassifizierung eines Untersuchungsobjekts mittels Aufnahmen (A1, A2) umfassend
- eine Erfassungseinheit (21), die so ausgebildet ist, dass sie optische Aufnahmen (A1) des Untersuchungsobjekts (3) erfasst,
- eine Analyseeinheit (27), die so ausgebildet ist, dass sie definierte Merkmale (CH1, CH2,...) des Untersuchungsobjekts (3) auf Basis einer Analyse der optischen Aufnahmen (A1) unter Verwendung eines maschinellen Lernverfahrens (L1) ermittelt und quantifiziert sowie
- eine Klassifizierungseinheit (24), die so ausgebildet ist, dass sie das Untersuchungsobjekt (3) hinsichtlich eines Klassifizierungskriteriums (CRT) auf Basis der quantifizierten Merkmale (SC1, SC2,...) unter Verwendung eines maschinellen Lernverfahrens (L2) klassifiziert.

12. Medizintechnisch bildgebende Modalität (1) mit einer Klassifizierungseinrichtung (20) nach Anspruch 11.

13. Computerprogrammprodukt mit einem Computerprogramm, welches direkt in eine Speichereinrichtung einer Klassifizierungseinrichtung (20) einer medizintechnisch bildgebenden Modalität (1) ladbar ist, mit Programmabschnitten, um alle Schritte eines Verfahrens nach einem der Ansprüche 1 bis 10 auszuführen, wenn das Computerprogramm in der Klassifizierungseinrichtung (20) der medizintechnisch bildgebenden Modalität (1) ausgeführt wird.

14. Computerlesbares Medium, auf welchem von einer Rechnereinheit (12) einlesbare und ausführbare Programmabschnitte gespeichert sind, um alle Schritte eines Verfahrens nach einem der Ansprüche 1 bis 10 auszuführen, wenn die Programmabschnitte von der Rechnereinheit (12) ausgeführt werden.
